# EUROPÄISCHE PATENTSCHRIFT

(11) **EP 1 950 287 B1**
(45) Veröffentlichungstag und Bekanntmachung des Hinweises auf die Patenterteilung: **26.08.2009**
(21) Anmeldenummer: 08152747.5
(22) Anmeldetag: 01.07.2004
(51) Int. Cl.: C12N 9/04, C12N 15/53, C12P 13/06

(54) **Varianten der 3-Phosphoglyceratdehydrogenase mit reduzierter Hemmung durch L-Serin und dafür codierende Gene**
3-Phosphoglycerate dehydrogenase variants whose inhibition by L-serine is reduced, and genes encoding them
Variants de la 3-phosphoglycerate deshydrogenase presentant une inhibition reduite par la serine, et genes codant pour ceux-ci

(30) Priorität: 10.07.2003 DE 10331291
(43) Veröffentlichungstag der Anmeldung: 30.07.2008
(62) Teilanmeldung aus: 07102448.3
(73) Patentinhaber: Wacker Chemie AG, 81737 München (DE)
(72) Erfinder: Maier, Thomas, Dr., 07743 Jena (DE); Flinspach, Renate, 83623 Dietramszell (DE)
(74) Vertreter: Potten, Holger

(56) Entgegenhaltungen:
- WO-A-93/12235
- WO-A-94/08031
- GRANT GREGORY A ET AL: "Probing the regulatory domain interface of D-3-phosphoglycerate dehydrogenase with engineered tryptophan residues" JOURNAL OF BIOLOGICAL CHEMISTRY, Bd. 273, Nr. 35, 28. August 1998 (1998-08-28), Seiten 22389-22394, XP002295411 ISSN: 0021-9258
- AL-RABIEE REGINA ET AL: "The mechanism of velocity modulated allosteric regulation in D-3-phosphoglycerate dehydrogenase: Cross-linking adjacent regulatory domains with engineered disulfides mimics effector binding" JOURNAL OF BIOLOGICAL CHEMISTRY, Bd. 271, Nr. 22, 1996, Seiten 13013-13017, XP002295412 ISSN: 0021-9258
- AL-RABIEE REGINA ET AL: "The mechanism of velocity modulated allosteric regulation in D-3-phosphoglycerate dehydrogenase: Site-directed mutagenesis of effector binding site residues" JOURNAL OF BIOLOGICAL CHEMISTRY, Bd. 271, Nr. 38, 1996, Seiten 23235-23238, XP002438700 ISSN: 0021-9258
- GRANT GREGORY A ET AL: "Specific interactions at the regulatory domain-substrate binding domain interface influence the cooperativity of inhibition and effector binding in Escherichia coli D-3-phosphoglycerate dehydrogenase" JOURNAL OF BIOLOGICAL CHEMISTRY, Bd. 276, Nr. 2, 12. Januar 2001 (2001-01-12), Seiten 1078-1083, XP002295413 ISSN: 0021-9258

## Beschreibung

Die Erfindung betrifft Varianten der 3-Phospoglyceratdehydrogenase mit reduzierter Hemmung durch L-Serin und dafür codierende Gene.

Die Herstellung der zwanzig natürlichen, proteinogenen Aminosäuren wird heutzutage vorwiegend durch Fermentation von Mikroorganismen bewerkstelligt. Dabei wird ausgenützt, dass Mikroorganismen über entsprechende Biosynthesewege zur Synthese der natürlichen Aminosäuren verfügen.

Solche Biosynthesewege unterliegen jedoch in Wildtyp-Stämmen einer strengen Kontrolle, die gewährleistet, dass die Aminosäuren nur zum Eigenbedarf der Zelle hergestellt werden. Ein wichtiger Kontrollmechanismus in vielen Biosynthesen ist beispielsweise das Phänomen der Feedback-Hemmung (oder Endprodukt-Hemmung). Dabei wird meist dasjenige Enzym eines Biosyntheseweges, das die einleitende enzymatische Reaktion dieses Biosynthesewegs katalysiert, durch das Endprodukt des Biosyntheseweges gehemmt. Die Hemmung findet meist durch eine allosterische Bindung des Endproduktes an das Enzym statt und bewirkt eine Konformationsänderung in einen inaktiven Zustand. So wird sichergestellt, dass bei einer Anhäufung des Endproduktes in der Zelle die weitere Synthese durch Hemmung des einleitenden Schrittes unterbunden wird.

Eine effiziente industrielle Produktion von Stoffwechselprodukten (wie z.B. Aminosäuren) ist deshalb nur möglich, wenn die Restriktionen durch Feedbackhemmung eines Stoffwechselweges aufgehoben werden können und damit Mikroorganismen verfügbar gemacht werden, die im Gegensatz zu Wildtyp-Organismen eine drastisch gesteigerte Produktionsleistung für die Herstellung des gewünschten Stoffwechselprodukts aufweisen.

Die Phosphoglycerat-Familie von Aminosäuren ist dadurch definiert, dass es sich um Aminosäuren handelt, die in ihrer Biosynthese von der 3-Phosphoglycerinsäure abgeleitet werden. Der natürliche Pfad des Stoffwechsels führt dabei zunächst über die Zwischenstufen 3-Phospohydroxypyruvat und 3-Phospho-L-serin zu L-Serin. L-Serin kann weiterhin zu Glycin bzw. über O-Acetyl-Serin zu L-Cystein umgesetzt werden. Auch L-Tryptophan ist zu dieser Gruppe zu zählen, da es sich ebenfalls in der Biosynthese von L-Serin ableitet. Ebenso sind unnatürliche Aminosäuren, die nach dem in US 2002/0039767 A1 beschrieben Verfahren hergestellt werden, der Phosphoglycerat-Familie zuzuordnen.

Verbindungen, die sich aus dem C1-Stoffwechsel ableiten, zeigen ebenfalls eine Abhängigkeit von der Biosynthese der Aminosäuren der Phosphoglycerat-Familie. Dies beruht auf der Tatsache, dass bei der Konversion von L-Serin zu Glycin Tetrahydrofolat als C1-Gruppenakzeptor fungiert und das beladende Tetrahydrofolat als zentraler Methylgruppendonor im C1-Stoffwechsel an vielen Biosynthesen (z.B. L-Methionin, Nukleotide, Pantothensäure, etc.) beteiligt ist. Erfindungsgemäß sind Verbindungen, die sich aus dem C1-Stoffwechsel ableiten somit vorzugsweise Verbindungen, die in ihrer Biosynthese von einer C1-Gruppenübertragung über Tetrahydrofolsäure abhängen.

Der einleitende Schritt der Biosynthese von Aminosäuren der Phosphoglycerat-Familie ist die Oxidation der D-3-Phosphoglycerinsäure zu 3-Phosphohydroxypyruvat und wird durch das Enzym 3-Phosphoglyceratdehydrogenase (PGD) [EC 1.1.1.95] katalysiert. Als Akzeptor für die bei der Reaktion entstehenden Reduktionsäquivalente dient NAD⁺, das zu NADH/H⁺ umgesetzt wird.

PGD-Enzyme sind aus verschiedensten Organismen bekannt (z.B. Rattus norvegicus, Arabidopsis thaliana, Escherichia coli, Bacillus subtilis). Dabei unterliegen die besser charakterisierten mikrobiellen Vertreter einer Feedback-Hemmung durch L-Serin.

Auf der Ebene der Aminosäuresequenz sind die mikrobiellen PGD-Enzyme im N-terminalen Teil (Aminosäure 1-340 der E. coli Sequenz) untereinander sehr ähnlich, wogegen der C-terminale Anteil nur geringe Ähnlichkeiten aufweist. Gerade in diesem C-terminalen Teil ist die regulatorische Domäne lokalisiert, die für die Serin-Hemmung verantwortlich ist (Peters-Wendisch et al., 2002, Appl. Microbiol. Biotechnol. 60:437-441).

Die am besten charakterisierte PGD ist diejenige aus Escherichia coli. Das Enzym wurde eingehend biochemisch untersucht (Dubrow & Pizer, 1977, J. Biol. Chem. 252, 1527-1538) und unterliegt einer allosterischen Feedback-Hemmung durch L-Serin mit einer Inhibitorkonstante Kᵢ von 5 µM.

Diese Feedback-Hemmung steht einer effizienten Produktion von Aminosäuren der Phosphoglycerat-Familie im Wege und war deshalb bereits Ziel molekularbiologischer Ansätze.

So wurden in der Schrift EP0620853A Varianten der Escherichia coli PGD mit verringerter Empfindlichkeit gegen Hemmung durch Serin beschrieben, die eine Modifikation in den C-terminalen 25% der Wildtyp-PGD, (d.h. Aminosäuren 307-410), bevorzugt eine Modifikation im Bereich der letzten 50 Reste (d.h. Aminosäuren 361-410) aufweisen. Die beschriebenen Mutanten wurden durch Linker-Mutagenese, d.h. durch einfache Nutzung von vorhandenen Restriktionsschnittstellen im E. coli serA-Gen und anschließendes Einsetzen von Oligonukleotid-Linkern einer Länge von 8-14 Basenpaaren erhalten.

Solche Linker-Mutagenesen sind jedoch meist problematisch, da durch den Einbau bzw. die Deletion von mehreren Resten die Struktur des Proteins sehr stark verändert wird und so die Gesamtaktivität oder Stabilität des Proteins negativ beeinflusst wird. Tatsächlich zeigen die meisten in EP0620853A beschriebenen Mutanten eine kaum nachweisbare Aktivität.

Auch in coryneformen Mikroorganismen wurden Mutagenesen am serA-Gen vorgenommen, die das Ziel einer Verringerung der Empfindlichkeit der PGD gegen Hemmung durch Serin erfüllen:
- Peters-Wendisch et al. (2002, Appl. Microbiol. Biotechnol. 60:437-441) beschreiben C-terminale Deletionen der PGD von Corynebacterium glutamicum. Auch hier führen die Deletionen zu teilweise starken Einbußen an Enzymaktivität.
- Die Anmeldung EP0943687A2 beschreibt einen Austausch des Glutaminsäure-Restes an Position 325 der PGD von Brevibacterium flavum. Dieser Rest liegt im Bezug zur Escherichia coli PGD in einem Alignment mit dem Algorithmus GAP des Programms GCG (GCG Wisconsin Package, Genetics Computer Group (GCG) Madison, Wisconsin) bereits in dem variablen C-terminalen Teil des Proteins und korreliert mit dem Asparaginrest 364 des Escherichia coli Proteins. Da diese Modifikation in dem variablen C-terminalen Teil der PGD liegen, sind keine Rückschlüsse auf das Escherichia coli Protein möglich.

Aus Grant Gregory A. et al., Journal of Biological Chemistry, Bd. 271, Nr. 35, 28. August 1998, Seiten 22389-22394 ist bekannt, dass die G349 W Varianten der E.coli PGD gegen eine Hemmung durch Serin eine wesentlich verringerte Empfindlichkeit aufweist. Al. Rabiee Regina et al., Journal of Biological Chemistry, Bd. 271, Nr. 22, 1996, Seiten 13013-13017 offenbart eine A359C, G349C Doppelmutante, welche dieselbe enzymatische Aktivität wie die E.coli-Wildtyp-PGD aufweist, jedoch eine um den Faktor 100 verringerte Empfindlichkeit gegen eine Hemmung durch Serin.

Aufgabe der vorliegenden Erfindung war es, eine Variante der PGD von Escherichia coli zur Verfügung zu stellen, die eine im Vergleich zur Escherichia coli-Wildtyp-PGD verringerte Empfindlichkeit gegen eine Hemmung durch Serin aufweist.

Die Aufgabe wird gelöst durch eine PGD mit einer Aminosäuresequenz, die der Aminosäuresequenz der Escherichia coli-Wildtyp-PGD (SEQ ID NO: 2) bis auf die Position 349 entspricht, dadurch gekennzeichnet, dass sich an Position 349 die Aminosäure E befindet.

Die Erfindung betrifft ferner eine DNS-Sequenz codierend für eine erfindungsgemäße PGD. Dieses serA-Allel unterscheidet sich vom Gen der Escherichia coli PGD (serA-Gen, SEQ ID NO: 1) dadurch, dass das Codon 349 für die natürliche Aminosäure E codiert.

PGD-Varianten, die einen Aminosäureaustausch an Position 349 besitzen, können mit Standardtechniken der Molekularbiologie erzeugt werden. Dazu werden an den entsprechenden Codons Mutationen in das für die PGD codierende serA-Gen eingebracht. Entsprechende Methoden zur Einführung von Mutationen an spezifischen Positionen innerhalb eines DNS-Fragmentes sind bekannt.

Als Ausgangsmaterial für die Mutagenese dient vorzugsweise die DNS des E. coli serA-Gens. Das zu mutierende serA-Gen kann chromosomal oder extrachromosomal codiert sein. Bevorzugt wird das serA-Gen jedoch durch Polymerase-Ketten-Reaktion amplifiziert und in einen Vektor kloniert. Durch Anwendung der vorgenannten Mutagenese-Methoden werden ein oder mehrere Nukleotide der DNS-Sequenz so verändert, dass die codierte PGD die Aminosäure E an Position 349 aufweist, wobei Position 1 das Startmethionin aus SEQ ID NO: 1 ist.

Diese Mutationen bewirken, dass die codierte PGD eine verminderte Empfindlichkeit gegen Hemmung durch L-Serin (=Feedback-Resistenz) besitzt. Dabei ist besonders vorteilhaft, dass die erfindungsgemäßen PGD-Varianten in Abwesenheit von L-Serin im Vergleich zur Wildtyp-PGD eine Aktivität von mehr als 10 %, vorzugsweise eine unveränderte Aktivität, aufweist.

Zur Bestimmung des Ausmaßes der Feedback-Resistenz einer erfindungsgemäßen PGD-Variante kann jede Methode benützt werden, die es erlaubt, die Aktivität des Enzyms in Anwesenheit von L-Serin zu bestimmen. Beispielsweise kann die Bestimmung der PGD-Aktivität in Anlehnung an die von McKitrick und Pizer (1980, J.Bacteriol. 141:235-245) beschriebene Methode erfolgen. Die Enzymaktivität wird anhand der Rückreaktion in einem Ansatz, der Phosphohydroxypyruvat und NADH/H⁺ enthält, gemessen. Die Reaktion wird durch Enzymzugabe gestartet und über die Abnahme der Extinktion bei 340 nm, die durch Oxidation des NADH/H⁺ hervorgerufen wird, in einem Spektralphotometer verfolgt. Die Hemmung der PGD-Aktivität wird in Anwesenheit verschiedener Konzentrationen von L-Serin im Reaktionsansatz getestet. Die katalytische Aktivität der verschiedenen PGD-Varianten wird in An- und Abwesenheit von L-Serin bestimmt und daraus die Inhibitorkonstante Kᵢ ermittelt. Der Kᵢ beschreibt diejenige Inhibitorkonzentration, bei welcher die Aktivität nur noch 50 % der Aktivität beträgt, die in Abwesenheit des Inhibitors bestimmt wurde.

Das erfindungsgemäße PGD-Enzym kann aufgrund seiner Feedback-Resistenz zur Herstellung von Aminosäuren der Phosphoglycerat-Familie oder von Verbindungen, die sich aus dem C1-Stoffwechsel ableiten, verwendet werden. Hierfür wird das erfindungsgemäße serA-Allel in einem Wirtsstamm exprimiert.

Die Expression des erfindungsgemäßen serA-Allels kann unter Kontrolle des eigenen, vor dem serA-Gen lokalisierten Promotors oder durch Verwendung anderer geeigneter Promotorsysteme, die dem Fachmann bekannt sind, erfolgen. Dabei kann sich das entsprechende Allel beispielsweise unter der Kontrolle eines solchen Promotors entweder in einer oder in mehreren Kopien auf dem Chromosom des Wirtsorganismus befinden. Die Strategien zur Integration von Genen in das Chromosom sind Stand der Technik. Bevorzugt wird das zu exprimierende serA-Allel jedoch in einen Vektor kloniert, vorzugsweise ein Plasmid.

Die Erfindung betrifft daher auch einen Vektor, **dadurch gekennzeichnet, dass** er ein erfindungsgemäßes serA-Allel unter funktioneller Kontrolle eines Promotors enthält.

Zur Klonierung des erfindungsgemäßen serA-Allels können Vektoren verwendet werden, die bereits genetische Elemente (z.B. konstitutive oder regulierbare Promotoren, Terminatoren) enthalten, die entweder eine andauernde oder eine kontrollierte, induzierbare Expression des für die PGD codierenden Gens ermöglichen. Außerdem befinden sich auf einem Expressionsvektor vorzugsweise andere regulatorische Elemente wie ribosomale Bindungsstellen und Terminationssequenzen sowie Sequenzen, die für selektive Marker und/oder Reporter-Gene codieren. Die Expression derartiger Selektionsmarker erleichtert die Identifizierung von Transformanten. Als Selektionsmarker geeignet sind Gene, die für eine Resistenz gegenüber z. B. Ampicillin, Tetracyclin, Chloramphenicol, Kanamycin oder anderen Antibiotika codieren. Wenn das erfindungsgemäße serA-Allel extrachromosomal repliziert werden soll, sollte der Plasmidvektor vorzugsweise einen Ursprungspunkt der Replikation enthalten. Besonders bevorzugt sind Plasmidvektoren wie beispielsweise die E. coli-Vektoren pACYC184, pUC18, pQE-70, pBR322, pSC101 und ihre Derivate. Als induzierbare Promotoren eignen sich beispielsweise der lac-, tac-, trc-, lambda PL, ara- oder tet-Promotor oder davon abgeleitete Sequenzen.

Des weiteren sind Plasmidvektoren besonders bevorzugt, die bereits ein Gen/Allel enthalten, dessen Einsatz ebenfalls zu einer Überproduktion von Aminosäuren der Phosphoglycerat-Familie bzw. von Verbindungen, die sich aus dem C1-Stoffwechsel ableiten, führt, wie beispielsweise für die Produktion von:
- L-Serin (z.B. serB-, serC-, Exportcarrier-Gen wie beschreiben in DE10044831A1)
- N-Acetyl-Serin, O-Acetyl-Serin, Cystin, Cystein oder Cysteinderivaten (z.B. cysE-Allele wie beschrieben in WO97/15673, Efflux-Gene wie beschrieben in EP0885962A1, cysB-Gen wie beschrieben in DE19949579C1, yfiK-Gen wie beschrieben in DE 10232930A)
- L-Tryptophan (z.B. trpE-Allele wie beschrieben in EP0662143A)
- Pantothensäure (z.B. wie beschrieben in WO02061108)

Solche Vektoren ermöglichen die direkte Herstellung von erfindungsgemäßen Mikroorganismenstämmen mit hoher Produktionsleistung aus einem beliebigen Mikroorganismenstamm, da ein solches Plasmid auch die Aufhebung von anderen Restriktionen des Stoffwechselweges in einem Mikroorganismus bewirkt.

Durch eine gängige Transformationsmethode (z.B. Elektroporation) werden die erfindungsgemäßen serA-Allel-haltigen Plasmide in Mikroorganismen eingebracht und beispielsweise mittels Antibiotika-Resistenz auf plasmid-tragende Klone selektiert.

Die Erfindung betrifft somit auch Verfahren zur Herstellung eines erfindungsgemäßen Mikroorganismenstammes, **dadurch gekennzeichnet, dass** in einen Mikroorganismenstamm ein erfindungsgemäßer Vektor eingebracht wird.

Es ist auch möglich Vektoren mit einem erfindungsgemäßen serA-Allel in Mikroorganismen einzubringen, die beispielsweise einzelne oder mehrere der oben genannten Gene/Allele bereits chromosomal exprimieren und bereits eine Überproduktion eines Stoffwechselprodukts aufweisen. In solchen Fällen kann durch das Einbringen eines erfindungsgemäßen serA-Allels die Produktionsleistung nochmals gesteigert werden.

Generell sind als Wirtsorganismus für erfindungsgemäße Vektoren alle Organismen geeignet, die den Biosyntheseweg für Aminosäuren der Phosphoglycerat-Familie aufweisen, rekombinanten Verfahren zugänglich sind und durch Fermentation kultivierbar sind. Solche Mikroorganismen können Pilze, Hefen oder Bakterien sein. Bevorzugt kommen Bakterien der phylogenetischen Gruppe der Eubacteria zum Einsatz. Besonders bevorzugt sind Mikroorganismen der Familie Enterobacteriaceae und insbesondere der Art Escherichia coli.

Die Erfindung betrifft somit ferner einen Mikroorganismenstamm, der zur fermentativen Herstellung von Aminosäuren der Phosphoglycerat-Familie oder deren Derivaten bzw. von Verbindungen, die sich aus dem C1 Stoffwechsel ableiten, geeignet ist, **dadurch gekennzeichnet, dass** er eine erfindungsgemäße PGD besitzt.

Ein weiterer Gegenstand der Erfindung ist die Herstellung von Aminosäuren der Phosphoglycerat-Familie bzw. von Verbindungen, die sich aus dem C1-Stoffwechsel ableiten, durch Kultivierung eines erfindungsgemäßen Mikroorganismenstammes.

Dazu wird der erfindungsgemäße Mikroorganismenstamm beispielsweise in einem Fermenter in einem Nährmedium kultiviert, das eine geeignete Kohlenstoff-, und eine geeignete Energiequelle, sowie andere Zusatzstoffe enthält.

Die während der Fermentation gebildeten Substanzen wie beispielsweise L-Phosphoserin, L-Serin, O-Acetyl-L-Serin, L-Cystein, Glycin, L-Tryptophan, 1,2,4-Triazol-2-yl-L-alanin, L-Methionin oder Pantothensäure können anschließend aufgereinigt werden.

Die folgenden Beispiele dienen der weiteren Erläuterung der Erfindung. Sämtliche eingesetzten molekularbiologischen Verfahren, wie Polymerase-Kettenreaktion, Isolierung und Reinigung von DNS, Modifikation von DNS durch Restriktionsenzyme, Klenow-Fragment und Ligase, Transformation etc. wurden in der dem Fachmann bekannten, in der Literatur beschriebenen oder von den jeweiligen Herstellern empfohlenen Art und Weise durchgeführt.

### Beispiel 1: Klonierung des serA-Gens

Das serA-Gen aus Escherichia coli Stamm W3110 (American Type Culture Collection, ATCC27325) wurde mit Hilfe der Polymerase-Ketten-Reaktion amplifiziert. Als spezifische Primer dienten die Oligonukleotide serA-fw: (SEQ ID NO: 3) und
serA-rev: (SEQ ID NO: 4)

Das resultierende DNS-Fragment wurde mit den Restriktionsenzymen NdeI und HindIII verdaut und die 5*'*-Überhänge mit Klenow-Enzym aufgefüllt. Anschließend wurde das DNS-Fragment mit Hilfe einer Agarose-Gelelektrophorese gereinigt und mit der GeneClean-Methode isoliert (GeneClean Kit BIO101 P.O.Box 2284 La Jolla, California, 92038-2284). Die Klonierung des so erhaltenen serA-Fragments erfolgte in den Expressionsvektor pQE-70 (Qiagen, Hilden, D). Hierfür wurde der Vektor zunächst mit SphI und BamHI geschnitten und der 3'-Überhang mit Klenow-Enzym abgedaut bzw. der 5'-Überhang mit Klenow-Enzym aufgefüllt. Anschließend wurde das Vektorfragment gereinigt und mit dem serA-Fragment ligiert. Der resultierende Vektor trägt die Bezeichnung pFL209. Nach der Verifizierung des Konstrukts durch Sequenzierung wurde der Escherichia coli Stamm JM109 (Stratagene, Amsterdam, NL) transformiert und entsprechende Transformanten mit Ampicillin selektiert. Der Bakterienstamm Escherichia coli JM109 / pFL209 wurde bei der DSMZ (Deutsche Sammlung für Mikroorganismen und Zellkulturen GmbH, D-38142 Braunschweig) unter der Nummer DSM 15628 gemäß Budapester Vertrag hinterlegt.

### Beispiel 2: Ortsgerichtete Mutagenese des serA-Gens

Die ortsspezifische Mutagenese an dem Codon 349 des serA-Gens wurde mittels einer inversen Polymerase-Ketten-Reaktion durchgeführt. Als Matrize diente der in Beispiel 1 beschriebene Vektor pFL209. Für die Mutagenese des Codons 349 wurden die Primer
serA40-mut (SEQ ID NO: 5)
   5'-GAA AAC CGT CCG NNN GTG CTA ACT GCG-3' N= G,A,T oder C
   und
serA40-rev (SEQ ID NO: 6)
   5'-GTG GAT GTG CAT CAG ACG-3'
   verwendet.

Das erhaltene PCR-Produkt wurde durch Ligation zirkularisiert und in den E. coli-Stamm JM109 transformiert. Durch Sequenzierung wurde schließlich die Mutation an Codon 349 bestimmt und die Korrektheit der übrigen Sequenz überprüft.

### Beispiel 3: Bestimmung der PGD-Aktivität und der Inhibitorkonstante Kᵢ

Zur Bestimmung von PGD-Enzymaktivitäten und des Einflusses von L-Serin auf die Aktivität wurden 100 ml LB-Medium (10 g/l Trypton, 5 g/l Hefeextrakt, 10 g/l NaCl), das zusätzlich 100 mg/l Ampicillin enthielt, mit einer 2 ml Übernachtkultur der Stämme mit den plasmidcodierten serA-Allelen beimpft und bei 30 °C und 150 rpm in einem Schüttler inkubiert. Bei einer optischen Dichte von 1,0 wurde die serA-Expression durch Zugabe von 0,4 mM Isopropyl-β-thiogalaktosid induziert und die Kultur für weitere 3 Stunden inkubiert. Die Zellen wurden anschließend durch Zentrifugation geerntet, gewaschen und in 2 ml Puffer (100 mM K-Phosphat pH 7,0; 10 mM MgCl₂; 1 mM Dithiothreitol) resuspendiert. Der Zellaufschluss erfolgte mittels einer French Press (Spectronic Instruments, Inc., Rochester, NY, USA) bei einem Druck von 18 000 psi. Die Rohextrakte wurden durch Zentrifugation bei 30 000 g geklärt und die PGD-Aktivität mit dem Test von McKitrick und Pizer (1980, J.Bacteriol. 141:235-245) bestimmt.
Die folgenden Tabellen zeigen die PGD-Aktivität verschiedener Mutanten, sowie die entsprechenden Inhibitorkonstanten Kᵢ.

**Tabelle 1: Mutation an Codon 349**

| **Allel** | **Mutation** | **Aktivität [units/mg]** | **Ki [mM]** |
|---|---|---|---|
| serA | Wildtyp | 0,05 | <0,1 |
| serA47 | G349E | 0,05 | 20 |

## Patentansprüche

1. 3-Phospoglyceratdehydrogenase (PGD) aufweisend eine im Vergleich zu einer Escherichia coli-Wildtyp-PGD verringerte Empfindlichkeit gegen eine Hemmung durch Serin mit einer Aminosäuresequenz, die der Escherichia coli-Wildtyp-PGD (SEQ ID NO: 2) bis auf die Position 349 entspricht, **dadurch gekennzeichnet, dass** sich an Position 349 die Aminosäure E befindet.

2. serA-Allel codierend für eine PGD gemäß Anspruch 1 **dadurch gekennzeichnet, dass** sie sich vom Gen der Escherichia coli PGD (serA-Gen, SEQ ID NO: 1) **dadurch** unterscheidet, dass das Codon 349 für die natürliche Aminosäure E codiert.

3. Vektor, **dadurch gekennzeichnet, dass** er ein serA-Allel gemäß Anspruch 2 unter funktioneller Kontrolle eines Promotors enthält.

4. Verfahren zur Herstellung eines Mikroorganismenstammes, **dadurch gekennzeichnet, dass** in einen Mikroorganismenstamm ein Vektor gemäß Anspruch 3 eingebracht wird.

5. Mikroorganismenstamm, der zur fermentativen Herstellung von Aminosäuren der Phosphoglycerat-Familie oder deren Derivaten bzw. von Verbindungen, die sich aus dem C1 Stoffwechsel ableiten, geeignet ist, **dadurch gekennzeichnet, dass** er eine PGD gemäß Anspruch 1 besitzt.

6. Verfahren zur Herstellung von Aminosäuren der Phosphoglycerat-Familie bzw. von Verbindungen, die sich aus dem C1 Stoffwechsel ableiten, durch Kultivierung eines Mikroorganismenstammes gemäß Anspruch 5.

7. Verwendung einer PGD gemäß Anspruch 1 zur Herstellung von Aminosäuren der Phosphoglycerat-Familie oder von Verbindungen, die sich aus dem C1-Stoffwechsel ableiten.

## Claims

1. 3-Phosphoglycerate dehydrogenase (PGD) which exhibits a susceptibility to inhibition by serine which is reduced as compared with that of an Escherichia coli wild-type PGD and which possesses an amino acid sequence which corresponds to the Escherichia coli wild-type PGD (SEQ ID NO: 2) except for position 349, **characterized in that** the amino acid E is present at position 349.

2. serA allele encoding a PGD according to Claim 1, **characterized in that** it differs from the Escherichia coli PGD gene (serA gene, SEQ ID NO: 1) **in that** codon 349 encodes the natural amino acid E.

3. Vector, **characterized in that** it contains a serA allele according to Claim 2 under the functional control of a promoter.

4. Method for producing a microorganism strain, **characterized in that** a vector according to Claim 3 is introduced into a microorganism strain.

5. Microorganism strain which is suitable for fermentatively producing amino acids of the phosphoglycerate family or their derivatives, or compounds which are derived from C1 metabolism, **characterized in that** said strain possesses a PGD according to Claim 1.

6. Method for producing amino acids of the phosphoglycerate family, or compounds which are derived from C1 metabolism, by culturing a microorganism strain according to Claim 5.

7. Use of a PGD according to Claim 1 for producing amino acids of the phosphoglycerate family, or compounds which are derived from C1 metabolism

## Revendications

1. 3-Phosphoglycérate déshydrogénase (PGD) présentant vis-à-vis d'une inhibition par la sérine une sensibilité réduite par rapport à celle d'une PGD de type sauvage d'Escherichia coli, ayant une séquence d'acides aminés qui correspond, jusqu'à la position 349, à celle de la PGD de type sauvage d'Escherichia coli (SEQ ID N° 2), **caractérisée en ce que** l'acide aminé E se trouve sur la position 349.

2. Allèle serA, codant pour une PGD selon la revendication 1, **caractérisé en ce qu'**il se distingue du gène de la PGD d'Escherichia coli (gène serA, SEQ ID N° 1) par le fait que le codon 349 code pour l'acide aminé naturel E.

3. Vecteur, **caractérisé en ce qu'**il contient un allèle serA selon la revendication 2, sous le contrôle fonctionnel d'un promoteur.

4. Procédé de fabrication d'une souche de microorganismes, **caractérisé en ce qu'**on introduit un vecteur selon la revendication 3 dans une souche de microorganismes.

5. Souche de microorganismes, qui convient à la fabrication, par fermentation, d'acides aminés de la famille des phosphoglycérates ou de leurs dérivés, ou de composés qui dérivent du métabolisme C1,
**caractérisée en ce qu'**elle possède une PGD selon la revendication 1.

6. Procédé de fabrication d'acides aminés de la famille des phosphoglycérates ou de composés qui dérivent du métabolisme C1, par culture d'une souche de microorganismes selon la revendication 5.

7. Utilisation d'une PGD selon la revendication 1 pour fabriquer des acides aminés de la famille des phosphoglycérates ou de composés qui dérivent du métabolisme C1.


### SEQUENCE LISTING

<110> Consortium fuer elektrochemische Industrie GmbH
<120> Varianten der 3-Phosphoglyceratdehydrogenase mit reduzierter Hemmung durch L-Serin und dafuer codierende Gene
<130> serA-Mutanten
<140>
   <141>
<160> 8
<170> PatentIn Ver. 2.0
<210> 1
   <211> 1233
   <212> DNA
   <213> Escherichia coli
<220>
   <221> CDS
   <222> (1)..(1230)
<400> 1
   atg gca aag gta tcg ctg gag aaa gac aag att aag ttt ctg ctg gta    48
   Met Ala Lys Val Ser Leu Glu Lys Asp Lys Ile Lys Phe Leu Leu Val
     1               5                  10                  15
   gaa ggc gtg cac caa aag gcg ctg gaa agc ctt cgt gca gct ggt tac 96
   Glu Gly Val His Gln Lys Ala Leu Glu Ser Leu Arg Ala Ala Gly Tyr
   20 25 30
   acc aac atc gaa ttt cac aaa ggc gcg ctg gat gat gaa caa tta aaa 144
   Thr Asn Ile Glu Phe His Lys Gly Ala Leu Asp Asp Glu Gln Leu Lys
   35 40 45
   gaa tcc atc cgc gat gcc cac ttc atc ggc ctg cga tcc cgt acc cat 192
   Glu Ser Ile Arg Asp Ala His Phe Ile Gly Leu Arg Ser Arg Thr His
   50 55 60
   ctg act gaa gac gtg atc aac gcc gca gaa aaa ctg gtc gct att ggc 240
   Leu Thr Glu Asp Val Ile Asn Ala Ala Glu Lys Leu Val Ala Ile Gly
   65 70 75 80
   tgt ttc tgt atc gga aca aac cag gtt gat ctg gat gcg gcg gca aag 288
   Cys Phe Cys Ile Gly Thr Asn Gln Val Asp Leu Asp Ala Ala Ala Lys
   85 90 95
   cgc ggg atc ccg gta ttt aac gca ccg ttc tca aat acg cgc tct gtt 336
   Arg Gly Ile Pro Val Phe Asn Ala Pro Phe Ser Asn Thr Arg Ser Val
   100 105 110
   gcg gag ctg gtg att ggc gaa ctg ctg ctg cta ttg cgc ggc gtg ccg 384
   Ala Glu Leu Val Ile Gly Glu Leu Leu Leu Leu Leu Arg Gly Val Pro
   115 120 125
   gaa gcc aat gct aaa gcg cac cgt ggc gtg tgg aac aaa ctg gcg gcg 432
   Glu Ala Asn Ala Lys Ala His Arg Gly Val Trp Asn Lys Leu Ala Ala
   130 135 140
   ggt tct ttt gaa gcg cgc ggc aaa aag ctg ggt atc atc ggc tac ggt 480
   Gly Ser Phe Glu Ala Arg Gly Lys Lys Leu Gly Ile Ile Gly Tyr Gly
   145 150 155 160
   cat att ggt acg caa ttg ggc att ctg gct gaa tcg ctg gga atg tat 528
   His Ile Gly Thr Gln Leu Gly Ile Leu Ala Glu Ser Leu Gly Met Tyr
   165 170 175
   gtt tac ttt tat gat att gaa aat aaa ctg ccg ctg ggc aac gcc act 576
   Val Tyr Phe Tyr Asp Ile Glu Asn Lys Leu Pro Leu Gly Asn Ala Thr
   180 185 190
   cag gta cag cat ctt tct gac ctg ctg aat atg agc gat gtg gtg agt 624
   Gln Val Gln His Leu Ser Asp Leu Leu Asn Met Ser Asp Val Val Ser
   195 200 205
   ctg cat gta cca gag aat ccg tcc acc aaa aat atg atg ggc gcg aaa 672
   Leu His Val Pro Glu Asn Pro Ser Thr Lys Asn Met Met Gly Ala Lys
   210 215 220
   gaa att tca cta atg aag ccc ggc tcg ctg ctg att aat gct tcg cgc 720
   Glu Ile Ser Leu Met Lys Pro Gly Ser Leu Leu Ile Asn Ala Ser Arg
   225 230 235 240
   ggt act gtg gtg gat att ccg gcg ctg tgt gat gcg ctg gcg agc aaa 768
   Gly Thr Val Val Asp Ile Pro Ala Leu Cys Asp Ala Leu Ala Ser Lys
   245 250 255
   cat ctg gcg ggg gcg gca atc gac gta ttc ccg acg gaa ccg gcg acc    816
   His Leu Ala Gly Ala Ala Ile Asp Val Phe Pro Thr Glu Pro Ala Thr
                   260                 265                 270
   aat agc gat cca ttt acc tct ccg ctg tgt gaa ttc gac aac gtc ctt    864
   Asn Ser Asp Pro Phe Thr Ser Pro Leu Cys Glu Phe Asp Asn Val Leu
               275                 280                 285
   ctg acg cca cac att ggc ggt tcg act cag gaa gcg cag gag aat atc    912
   Leu Thr Pro His Ile Gly Gly Ser Thr Gln Glu Ala Gln Glu Asn Ile
           290                 295                 300
   ggc ctg gaa gtt gcg ggt aaa ttg atc aag tat tct gac aat ggc tca    960
   Gly Leu Glu Val Ala Gly Lys Leu Ile Lys Tyr Ser Asp Asn Gly Ser
   305                 310                 315                 320
   acg ctc tct gcg gtg aac ttc ccg gaa gtc tcg ctg cca ctg cac ggt    1008
   Thr Leu Ser Ala Val Asn Phe Pro Glu Val Ser Leu Pro Leu His Gly
                       325                 330                 335
   ggg cgt cgt ctg atg cac atc cac gaa aac cgt ccg ggc gtg cta act    1056
   Gly Arg Arg Leu Met His Ile His Glu Asn Arg Pro Gly Val Leu Thr
                   340                 345                 350
   gcg ctg aac aaa atc ttc gcc gag cag ggc gtc aac atc gcc gcg caa    1104
   Ala Leu Asn Lys Ile Phe Ala Glu Gln Gly Val Asn Ile Ala Ala Gln
               355                 360                 365
   tat ctg caa act tcc gcc cag atg ggt tat gtg gtt att gat att gaa    1152
   Tyr Leu Gln Thr Ser Ala Gln Met Gly Tyr Val Val Ile Asp Ile Glu
   370 375 380
   gcc gac gaa gac gtt gcc gaa aaa gcg ctg cag gca atg aaa gct att 1200
   Ala Asp Glu Asp Val Ala Glu Lys Ala Leu Gln Ala Met Lys Ala Ile
   385 390 395 400
   ccg ggt acc att cgc gcc cgt ctg ctg tac taa 1233
   Pro Gly Thr Ile Arg Ala Arg Leu Leu Tyr
   405 410
<210> 2
   <211> 410
   <212> PRT
   <213> Escherichia coli
<400> 2
   Met Ala Lys Val Ser Leu Glu Lys Asp Lys Ile Lys Phe Leu Leu Val
   1 5 10 15
   Glu Gly Val His Gln Lys Ala Leu Glu Ser Leu Arg Ala Ala Gly Tyr
   20 25 30
   Thr Asn Ile Glu Phe His Lys Gly Ala Leu Asp Asp Glu Gln Leu Lys
   35 40 45
   Glu Ser Ile Arg Asp Ala His Phe Ile Gly Leu Arg Ser Arg Thr His
   50 55 60
   Leu Thr Glu Asp Val Ile Asn Ala Ala Glu Lys Leu Val Ala Ile Gly
   65 70 75 80
   Cys Phe Cys Ile Gly Thr Asn Gln Val Asp Leu Asp Ala Ala Ala Lys
   85 90 95
   Arg Gly Ile Pro Val Phe Asn Ala Pro Phe Ser Asn Thr Arg Ser Val
   100 105 110
   Ala Glu Leu Val Ile Gly Glu Leu Leu Leu Leu Leu Arg Gly Val Pro
   115 120 125
   Glu Ala Asn Ala Lys Ala His Arg Gly Val Trp Asn Lys Leu Ala Ala
   130 135 140
   Gly Ser Phe Glu Ala Arg Gly Lys Lys Leu Gly Ile Ile Gly Tyr Gly
   145 150 155 160
   His Ile Gly Thr Gln Leu Gly Ile Leu Ala Glu Ser Leu Gly Met Tyr
   165 170 175
   Val Tyr Phe Tyr Asp Ile Glu Asn Lys Leu Pro Leu Gly Asn Ala Thr
   180 185 190
   Gln Val Gln His Leu Ser Asp Leu Leu Asn Met Ser Asp Val Val Ser
   195 200 205
   Leu His Val Pro Glu Asn Pro Ser Thr Lys Asn Met Met Gly Ala Lys
   210 215 220
   Glu Ile Ser Leu Met Lys Pro Gly Ser Leu Leu Ile Asn Ala Ser Arg
   225 230 235 240
   Gly Thr Val Val Asp Ile Pro Ala Leu Cys Asp Ala Leu Ala Ser Lys
   245 250 255
   His Leu Ala Gly Ala Ala Ile Asp Val Phe Pro Thr Glu Pro Ala Thr
   260 265 270
   Asn Ser Asp Pro Phe Thr Ser Pro Leu Cys Glu Phe Asp Asn Val Leu
   275 280 285
   Leu Thr Pro His Ile Gly Gly Ser Thr Gln Glu Ala Gln Glu Asn Ile
   290 295 300
   Gly Leu Glu Val Ala Gly Lys Leu Ile Lys Tyr Ser Asp Asn Gly Ser
   305 310 315 320
   Thr Leu Ser Ala Val Asn Phe Pro Glu Val Ser Leu Pro Leu His Gly
   325 330 335
   Gly Arg Arg Leu Met His Ile His Glu Asn Arg Pro Gly Val Leu Thr
   340 345 350
   Ala Leu Asn Lys Ile Phe Ala Glu Gln Gly Val Asn Ile Ala Ala Gln
   355 360 365
   Tyr Leu Gln Thr Ser Ala Gln Met Gly Tyr Val Val Ile Asp Ile Glu
   370 375 380
   Ala Asp Glu Asp Val Ala Glu Lys Ala Leu Gln Ala Met Lys Ala Ile
   385 390 395 400
   Pro Gly Thr Ile Arg Ala Arg Leu Leu Tyr
   405 410
<210> 3
   <211> 30
   <212> DNA
   <213> Artificial Sequence
<220>
   <223> Primer for PCR
<400> 3
   gaattccata tggcaaaggt atcgctggag 30
<210> 4
   <211> 30
   <212> DNA
   <213> Artificial Sequence
<220>
   <223> Primer for PCR
<400> 4
   agaaagcttt tattagtaca gcagacgggc 30
<210> 5
   <211> 27
   <212> DNA
   <213> Artificial Sequence
<220>
   <223> Primer for PCR
<400> 5
   gaaaaccgtc cgnnngtgct aactgcg 27
<210> 6
   <211> 18
   <212> DNA
   <213> Artificial Sequence
<220>
   <223> Primer for PCR
<400> 6
   gtggatgtgc atcagacg 18